# EUROPEAN PATENT APPLICATION

(11) **EP 4 260 982 A1**
(43) Date of publication of application: **18.10.2023**
(21) Application number: 22382344.4
(22) Date of filing: 11.04.2022
(51) Int. Cl.: B25B 15/00, F16B 23/00

(54) **SCREW, SCREWDRIVER AND SCREW FASTENING SET**

(71) Applicant: Bonastre Biomed, SL, 08750 Molins de Rei (ES)
(72) Inventor: MARTINEZ ROIG, Sergi, 08750 Molins de Rei (ES)
(74) Representative: Torner, Juncosa I Associats, SL

(57) **Abstract**

Screw, screwdriver and screw fastening set, the screwdriver (1) comprising a screw engaging configuration (10) with a core (11) surrounded by a number of protruding longitudinal ribs (12) elongated in the longitudinal driver axis (DA) direction and radially distributed around the core (11) in radial symmetry, and wherein successive transversal cross-sections of the core (11), perpendicular to the longitudinal driver axis (DA), have a regular polygonal shape, longitudinal cross-sections of the core (11), coincident with the longitudinal driver axis (DA), have a convex shape, and each protruding longitudinal rib (12) is connected to the core (11) on an edge between two adjacent arched convex surfaces of the core, a central portion (13) of each side of the regular polygonal shape being exposed for coupling into a complementary flat surface of a driver engaging configuration of the screw, providing friction-retention between the screw and the screwdriver.

## Description

### Technical field

The present invention is directed to a screw, to a screwdriver for fixing screws at an angle and also to a screw fixing set comprising said screw and the complementary screwdriver, the screw and complementary screwdriver being adapted to produce releasable friction-attachment between the screw and the screwdriver.

The screw and screwdriver are preferably aimed for dental applications such as fixing a dental implant, or other medical applications, but other industrial applications are also envisaged.

### State of the Art

Fastening sets comprising screws and complementary screwdrivers which allow for fastening at an angle, are known for example by a hexagonal ball screwdriver, with a screw engaging configuration on its head having a regular polygonal transversal cross-section and a circular longitudinal cross-section.

Another well-known alternative for fastening at an angle is a hexa-lobular screwdriver with an screw engaging configuration on its head having a spherical core surrounded with several protruding ribs distributed in radial symmetry, i.e. evenly distributed around the central spherical core.

In the hexagonal ball solution, each surface of the polygonal head of the screwdriver engages with a complementary surface of the screw transmitting torque, but this configuration concentrate stress and wear on the edges of the polygonal head, which end up rounding off the polygonal head reducing its functionality.

In the hexa-lobular ball solution, the protruding ribs engage with complementary grooves of the screw, transmitting the torque through said protruding ribs, reducing or eliminating the wear problem, but the shape of those ribs does not allow for a precise fitting. Document EP2932937B1 describes a screw fastening set for dental applications with that hexa-lobular ball solution.

It is also known to provide an attachment between the screw and the screwdriver by magnetic means, but for some applications, especially medical applications, screws are preferably made of non-ferromagnetic materials, such titanium. In those cases, a nonmagnetic attachment between the screw and the screwdriver, such a friction-attachment obtained by a precise fitting between the screwdriver and the screw, is required for the insertion and extraction of the screw into/from the installation position, which in the medical or dental applications is within the patient's body or mouth.

None of the well-known solutions described above provide an attachment between the screw and the screwdriver, while avoiding an accelerate wear of the screwdriver.

Some available products provide a friction-attachment between the screw and the screwdriver but not allow for fastening the screw with the screwdriver at an angle. In certain applications the access to the final position of the screw is limited and the possibility for fastening the screw at an angle is required.

The present invention provides a solution to the above and other problems.

### Brief description of the invention

According to a first aspect, the present invention is directed to a screwdriver, for example for medical or dental applications, for fastening screws.

The proposed screwdriver comprises, in a manner already known, an elongated driver body, elongated in a longitudinal driver axis direction, with a screw engaging configuration on a distal end thereof.

The screw engaging configuration of the proposed screwdriver is intended to be coupled to a driver engaging configuration of a complementary screw and comprises a core surrounded by a number of protruding longitudinal ribs elongated in the longitudinal driver axis direction and radially distributed around the core, preferably in radial symmetry, defining a lobular screw engaging configuration.

According to that, the screw engaging configuration comprises a solid core surrounded by a number of longitudinal ribs, each protruding outwardly from the core, in a radial direction regarding the longitudinal driver axis direction and elongated towards the distal end tip of the screwdriver. Those longitudinal ribs are regularly distributed around the core.

This configuration is already known in the sector and is typically known as a hexa-lobular ball configuration or a Torx ball configuration.

Each of the protruding longitudinal ribs provide torque transmission between the screwdriver and the screw, whatever the angle between the screwdriver and the screw up to an angle of 45°, 30° or preferably up to an angle of 25°.

The present invention further proposes the screwdriver having the following features, which are not known:
- successive transversal cross-sections of the core, perpendicular to the longitudinal driver axis, have a regular polygonal shape, and longitudinal cross-sections of the core, coincident with the longitudinal driver axis, have a convex shape, defining a core (11) with several adjacent arched convex surfaces connected through arched edges; and
- each protruding longitudinal rib is connected to the core on one arched edge between two adjacent arched convex surfaces of the core, or on one arched convex surface of the core, a central portion of at least some of the arched convex surfaces of the core being exposed.

According to that, the core is a solid core with several identical adjacent arched convex surfaces connected through convex edges, each of said arched convex surfaces having a straight transversal cross-section and a convex longitudinal cross-section. Those identical arched convex surfaces determine that the successive transversal cross-sections of the core have a regular polygonal shape.

It will be understood that a regular polygonal shape is an equilateral (all sides have the same longitude) and equiangular (all angles between sides are equal) polygon with any number of sides above two. Said regular polygonal shape can be, for example, an equilateral triangle, a square, a pentagon, a hexagon, heptagon, octagon, etc.

Each longitudinal rib is connected to the core on one of said arched edges, covering it, between identical adjacent arched convex surfaces of the core, letting exposed a central portion of at least some of said arched convex surfaces of the core between two adjacent longitudinal ribs.

All the arched edges of the core may include a longitudinal rib thereon, but alternatively only some of the arched edges, for example alterne arched edges, may include longitudinal ribs thereon, the remaining arched edges being exposed.

Alternatively, the protruding ribs are attached not on the arched edges but on the center of the arched convex surfaces of the core, some of the arched convex surfaces, for example alterne arched convex surfaces, not including protruding ribs but the central exposed portions.

Each of the exposed central portions of the arched convex surfaces of the core, with a straight transversal cross-section and a convex longitudinal cross-section, provide a friction-coupling surface with a complementary surface of a driver engaging configuration of a screw, whatever the angle between the screwdriver and the screw up to an angle of 45°, 30° or preferably up to an angle of 25°.

Virtually, all the torque transmission between the screwdriver and the screw is conducted through the longitudinal ribs, so the exposed central portions of the surfaces of the core virtually do not transmit torque. This reduces the stress and the wear of said exposed central portions of the surfaces of the core, maintaining the friction-coupling surface undamaged for longer time.

According to a second aspect, the present invention is directed to a screw, preferably a screw made of a non-ferromagnetic material for medical or dental applications.

The proposed screw comprises, in a manner already known in the state of the art, an elongated screw body, elongated in a longitudinal screw axis direction, with a driver engaging configuration on a proximal end thereof, and with a threaded region on a distal end thereof.

The driver engaging configuration comprises a central socket surrounded by a number of longitudinal grooves elongated in the longitudinal screw axis direction and radially distributed around the central socket in radial symmetry, defining a lobular driver engaging configuration.

According to that, the driver engaging configuration comprises a central socket surrounded by a number of longitudinal grooves, each projecting outwardly from the central socket, in a radial direction regarding the longitudinal screw axis direction and elongated towards the distal end of the screw. Those longitudinal grooves are regularly distributed around the central socket.

This configuration is already known in the sector and is typically known as a Torx configuration.

Each of the longitudinal grooves provides torque transmission between the screwdriver and the screw, whatever the angle between the screwdriver and the screw up to an angle of 45°, 30° or preferably up to an angle of 25°.

The present invention further proposes the screw having the following features, which are not known:
- successive transversal cross-sections of the central socket, perpendicular to the longitudinal screw axis, have a regular polygonal shape with a diminishing size towards the proximal end of the screw, defining a tapering polygonal socked; and
- each longitudinal groove is defined between two adjacent surfaces of the tapering polygonal socket, or on one of said surfaces of the tapering polygonal socket, a central portion of each of said surfaces of the tapering polygonal socket being exposed.

According to that, the central socket is an empty housing for the screw engaging configuration of the screwdriver, accessible through a mouth concentric with the longitudinal screw axis.

The central socket is surrounded by several identical adjacent converging surfaces, surrounding the longitudinal screw axis, defining a regular polygonal shape, for example, an equilateral triangle, a square, a pentagon, a hexagon, heptagon, octagon, etc.

Each of said surfaces surrounding the central socket has a straight transversal cross-section, complementary to the straight transversal cross-section arched convex surfaces of the screw engaging configuration of the screwdriver.

Also, the central socket is tapered towards the distal end of the screw, having a reducing size away from the mouth. Said tapered central socket allows self-centering and friction-locking between the screw engaging configuration of the screwdriver and the driver engaging configuration of the screw.

Each longitudinal groove is defined on an edge between two adjacent surfaces of the tapered central socket, letting exposed a central portion of each of said surfaces of the tapered central socket between adjacent longitudinal ribs.

Alternatively, each longitudinal groove can be defined on one surface of the tapered central socket, at least some of the surfaces of the tapered central socket being devoid of longitudinal grooves, letting the central portion of said surface exposed, at least on some of the surfaces of the tapered central socket.

Each of the exposed central portions of the surfaces surrounding the tapered central socket, with a straight transversal cross-section, provide a friction-coupling surface with a complementary surface of the screw engaging configuration of a screwdriver, whatever the angle between the screwdriver and the screw up to an angle of 45°, 30° or preferably up to an angle of 25°.

Also, the combination of the longitudinal grooves and the regular polygonal shaped tapered central socket allow the fastening and unfastening of the screw not only through the screwdriver described above, but also using a normal Torx screwdriver which include the longitudinal ribs, complementary to the longitudinal grooves, but not the proposed friction-coupling surfaces on the core, or with a regular hex screwdriver or a hex ball screwdriver, which include the regular polygonal shaped cross-section core but devoid of longitudinal ribs, which will produce accelerated wear. According to that, when the Torx screwdriver is used, no friction-coupling between the screw and the screwdriver is obtained, and when a hex ball screwdriver is used the friction-coupling surfaces of the tapered central socket of the screw will transmit the torque and will suffer stress and accelerated wear.

In order to obtain said effect, preferably the longitudinal grooves of the lobular driver engaging configuration, but not the surfaces of the tapered central socket, are according to the standard ISO 10664, preferably ISO 10664:2005, which define the TORX standard.

The surfaces of the tapered central socket will be compatible with a polygonal screwdriver, such a hexagonal screwdriver under the standard of the dental industry known as hex. E/C, for example of between 1.20mm and 1.33mm.

The proposed surfaces of the tapered central socket do not interfere with a screwdriver according to ISO 10664 which protruding ribs are complementary with the longitudinal grooves of the proposed of the lobular driver engaging configuration, because said lobular driver engaging configuration circumscribes the shape defined by the standard ISO 10664. Accordingly, the protruding ribs of the screwdriver can be also according to the standard ISO 10664.

Those problems existing when different screwdrivers than the proposed one are used, do not exist when the proposed screwdriver is used, because virtually, all the torque transmission between the screwdriver and the screw is conducted through the longitudinal ribs, so the exposed central portions of the surfaces of the tapered central socket virtually do not transmit torque when the proposed screwdriver is used, reducing the stress and the wear of said exposed central portions of the surfaces of the tapered central socket, maintaining the friction-coupling surface undamaged for longer time.

According to a third aspect, the present invention is directed to a screw fastening set comprising the screwdriver described above and the screw described above.

The screwdriver and the screw of the same set will have regular polygonal shaped transversal cross-sections with the same number of sides, and the size of the biggest transversal cross-sections of the regular polygonal shaped core of the screw engaging configuration will be comprised between the biggest and the smallest sizes of the successive transversal cross-sections of the tapering central socket, providing friction-retention between the screw and driver engaging configurations when coupled.

According to one embodiment, the regular polygonal shaped transversal cross-sections of each the screwdriver and of the screw will have at least four sides.

Other features of the invention appear from the following detailed description of an embodiment.

### Brief description of the Figures

The foregoing and other advantages and features will be more fully understood from the following detailed description of an embodiment with reference to the accompanying drawings, to be taken in an illustrative and non-limitative manner, in which:
Fig. 1 shows a perspective view of the screw engaging configuration of the distal end of the screwdriver;
Fig. 2 shows a plan view of the screw engaging configuration shown on Fig. 1;
Fig. 3 shows a lateral view of the screw engaging configuration shown on Fig. 1;
Fig. 4A, 4B and 4C shows first, second and third transversal cross-sections of the screw engaging configuration shown on Fig. 1, for three successive transversal planes indicated in Fig. 3;
Fig. 5 shows a perspective view of a screw complementary to the screwdriver of Fig. 1;
Fig. 6 shows a plan view of the driver engaging configuration of the screw shown on Fig. 5;
Fig. 7 shows a longitudinal cross-sectional view of the driver engaging configuration shown on Fig. 5;

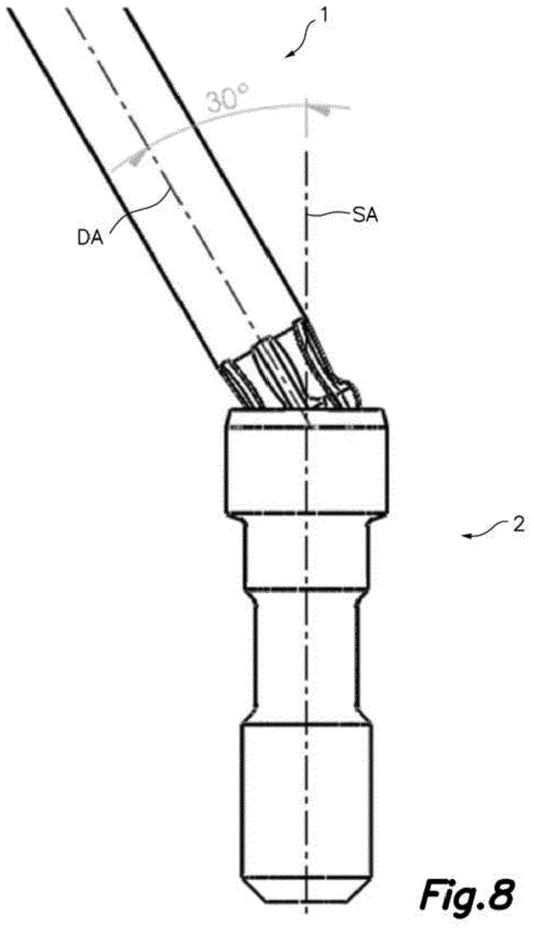

Fig. 8 shows a lateral view of the screw fastening set comprising a simplified view of the screw shown on Fig. 5 coupled at an angle with the screwdriver shown in Fig. 1;

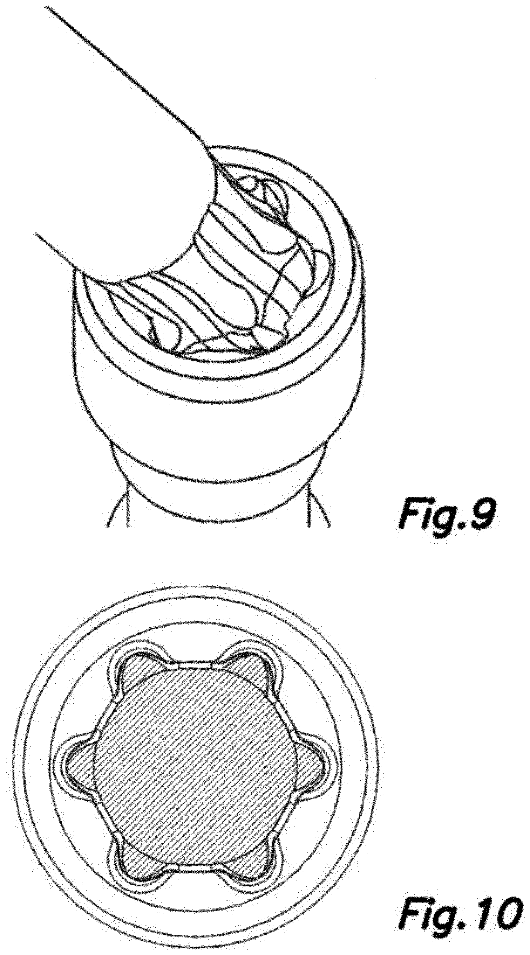

Fig. 9 shows a perspective view of the screw fastening set shown in Fig. 8;
Fig. 10 shows a transversal cross-section of the screw engaging configuration of the screwdriver shown on Fig. 1 coupled to the driver engaging configuration of the screw shown in Fig. 5;
Fig. 11 shows plan views of different alternative embodiments of the screw;

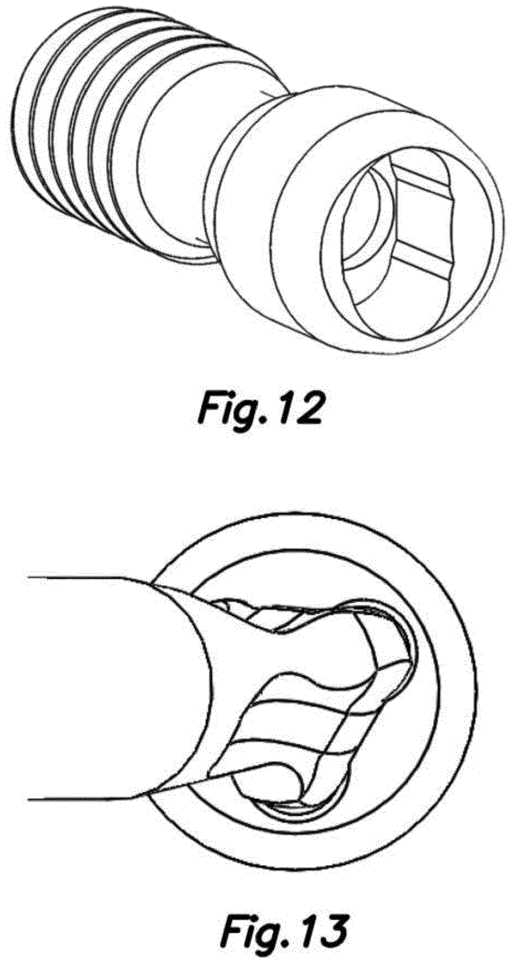

Fig. 12 shows a perspective view of an alternative embodiment of the screw with two longitudinal grooves on opposed surfaces of a quadrangular tapered central socket, letting two surfaces thereof exposed;
Fig. 13 shows a perspective view of an alternative embodiment of the screw fastening set with a screwdriver with three protruding ribs coupled to the complementary screw;
Fig. 14A to 14E shows a side view and a front view of the screw engaging configuration of the screwdriver according to different embodiments;
Figs. 15A to 15E shows a side view and a front view of the driver engaging configuration of the screw according to different embodiments;
Fig. 16 shows a front view of the proposed screw with a cross-sectioned screw engaging configuration of the proposed screwdriver inserted therein, and a lateral view thereof;
Fig. 17 shows a front view of the proposed screw with a cross-sectioned screw engaging configuration of a straight hexagonal screwdriver under the standard of the dental industry known as hex. E/C inserted therein, and a lateral view thereof;
Fig. 18 shows a front view of the proposed screw with a cross-sectioned screw engaging configuration of a straight hexagonal screwdriver under the standard of the dental industry known as Torx inserted therein, and a lateral view thereof;
Fig. 19 shows a front view of the proposed screw with a cross-sectioned screw engaging configuration of a straight hexagonal screwdriver under the standard of the dental industry known as six node security inserted therein, and a lateral view thereof.

### Detailed description of an embodiment

The foregoing and other advantages and features will be more fully understood from the following detailed description of an embodiment with reference to the accompanying drawings, to be taken in an illustrative and not limitative way.

According to the preferred embodiment, the proposed screwdriver 1 has an elongated driver body, typically a bar or a tube concentric with a longitudinal driver axis DA, with a screw engaging configuration 10 on a distal end thereof.

The screw engaging configuration 10 is intended to be engaged with a complementary driver engaging configuration 20 of the screw 2.

The proposed screw engaging configuration 10 comprises a core 11 surrounded by a number of protruding longitudinal ribs 12 distributed around the core in radial symmetry. Each of the longitudinal ribs 12 is elongated in the direction of the longitudinal driver axis DA, defining a lobular screw engaging configuration.

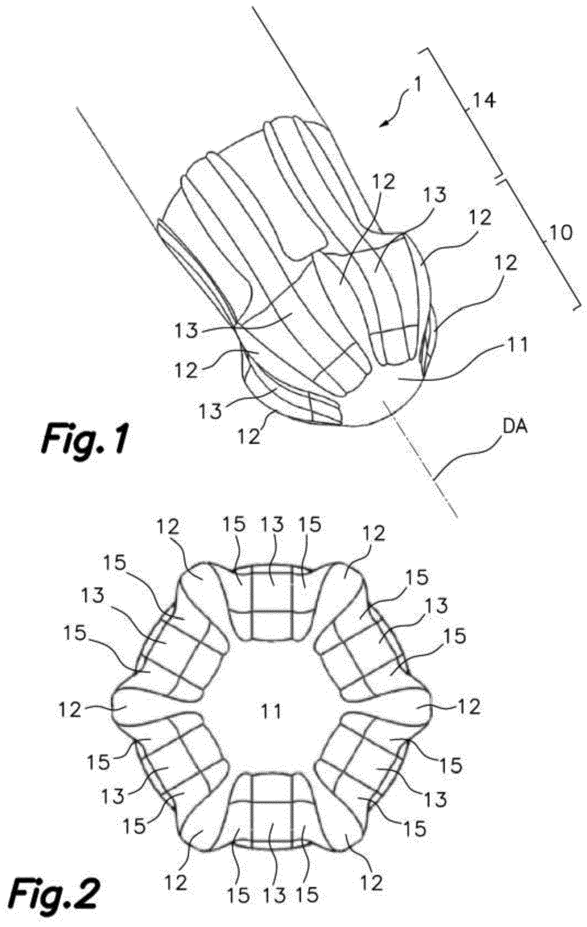

According to the preferred embodiment shown in Figs. 1, 2, 3, 4A, 4B and 4C the screw engaging configuration 10 comprises 6 longitudinal ribs 12, but a different number of longitudinal ribs 12 are also possible.

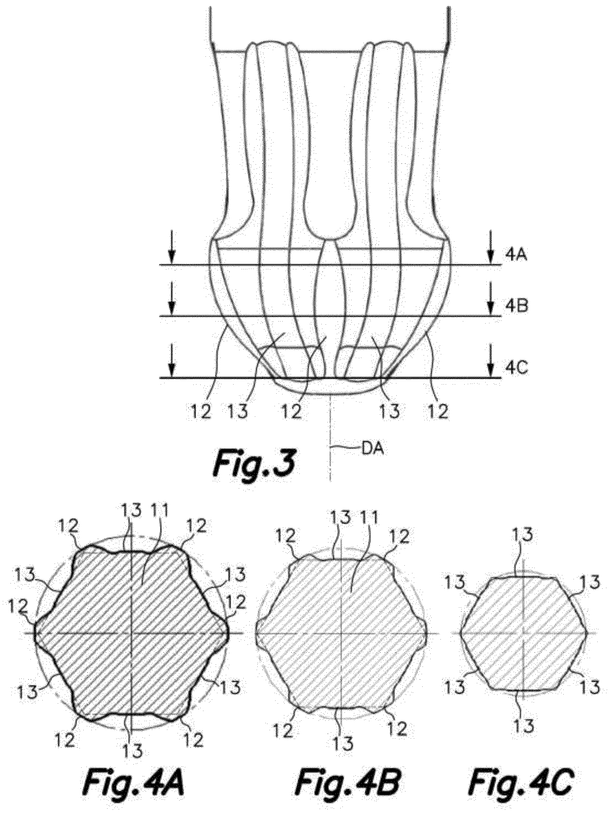

Figs. 4A, 4B and 4C show successive transversal cross-sections of the screw engaging configuration 10, perpendicular to the longitudinal driver axis DA, according to a first embodiment, showing the section of both the core 11 and the surrounding longitudinal ribs 12. On those transversal cross-sections, it is clear that the core 11 has a regular polygonal shape, in this example a hexagonal shape, with the longitudinal ribs 12 protruding from the edges of said regular polygonal shape, leaving exposed a central portion 13 of each side of the regular polygonal shape.

The longitudinal cross-sections of the core 11, coincident with the longitudinal driver axis DA, have a convex shape, as can be seen on Fig. 1.

According to the above, each arched convex surface of the core 11 is defined by a surface of zero Gaussian curvature, i.e. a curvature in a single direction which can be obtained by curving a flat surface without producing distortions on said surface.

Preferably, also the longitudinal cross-sections of the longitudinal ribs 12, protruding from the core 11, have a convex shape.

The convex shape of the longitudinal cross-section of the core 11, and/or of the longitudinal cross-section of the longitudinal ribs 12 protruding from the core 11, can be substantially semicircular shaped and/or substantially pointed arch shaped.

According to a preferred embodiment, the core 11 is connected to the elongated driver body through a slender portion 14 thinner than the core 11, which increases the allowable connection angle between the screw engaging configuration and the driver engaging configuration.

Successive transversal cross-sections of the slender portion 14, perpendicular to the longitudinal driver axis DA, will have the same regular polygonal shape than the core 11, and the longitudinal cross-sections of the slender portion 14, coincident with the longitudinal driver axis DA, will have a concave shape, defining a slender portion 14 with several adjacent arched concave surfaces.

The longitudinal ribs 12 can also protrude from the slender portion 14. In this case, the longitudinal cross-section of the longitudinal ribs 12, protruding from the slender portion 14, will be concave shaped.

According to a preferred embodiment, each longitudinal rib 12 is bordered by two longitudinal grooves 15 engraved in the core 11, the exposed central portion 13 of each arched convex surface of the core 11 being delimited between two of said longitudinal grooves 15.

The screw engaging configuration 10 of the screwdriver 1 may have at a distal end tip thereof, a flattened end devoid of elongated ribs 12.

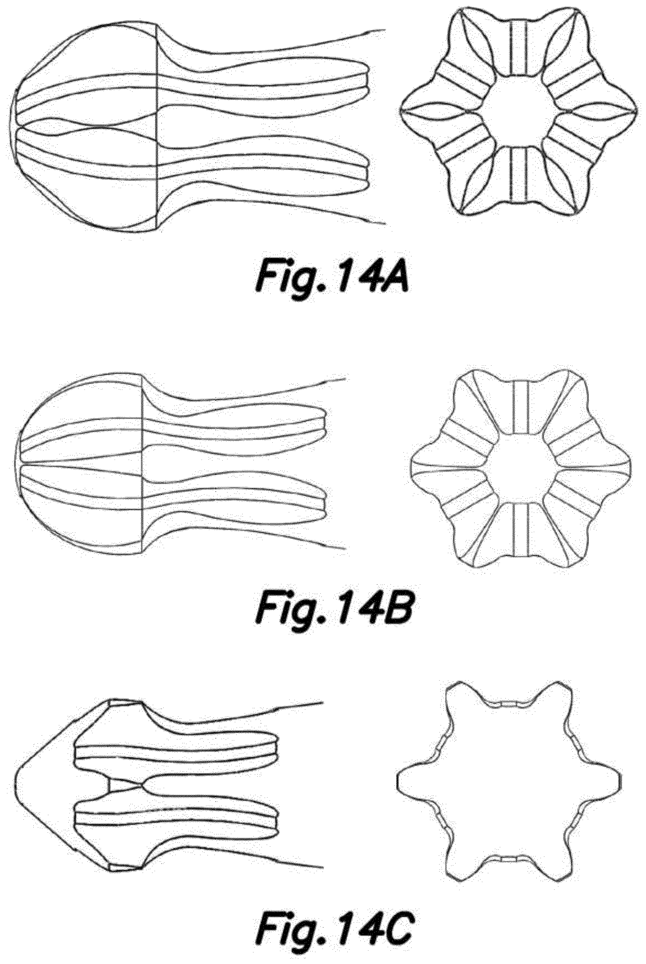

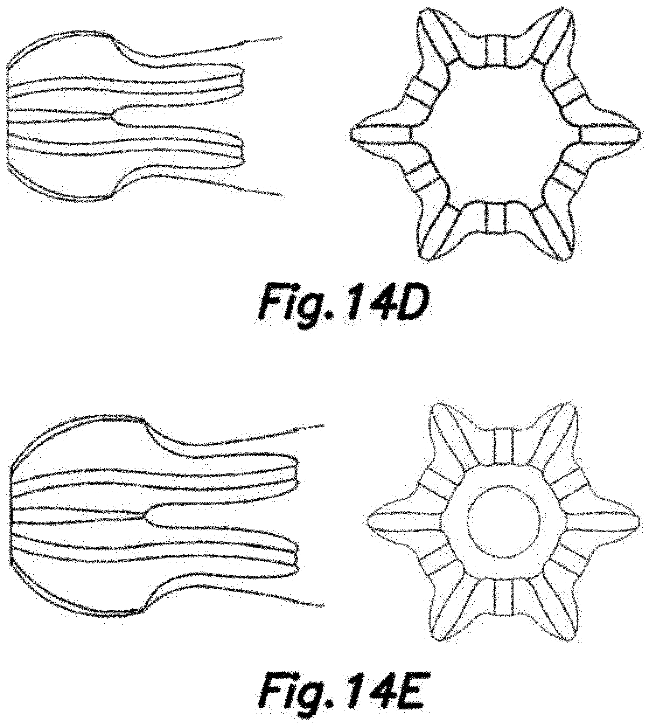

Figs. 14A to 14E shown different embodiments of the screw engaging configuration with different end tips. For example, Figs. 14D and 14E sown and end tip are completely flat, Figs. 14A and 14B shown a smooth and slightly convex end tip, and Fig. 14C shows a pointy rounded end tip covering up to a third of the screw engaging configuration.

Preferably, the height of the longitudinal ribs 12 from the core 11 diminishes towards the distal end tip of the screwdriver 1, typically reaching a zero height at the distal end tip of the screw engaging configuration 10.

The width of the longitudinal ribs 12 can also diminish towards the distal end tip of the screwdriver 1, optionally reaching zero width at the distal end tip of the screw engaging configuration 10.

Each longitudinal rib 12 may have a flattened external surface in the area furthest away from the core. Said flattened external surface may also have a variable width towards the distal end tip of the screwdriver 1.

According to the embodiment shown in Fig. 1 the width of each of the flattened external surfaces firstly increases and later decreases towards the distal end of the end tip, with a maximum width at a central portion of the correspondent longitudinal rib.

According to the embodiment shown in Fig. 14B the width of each of the flattened external surfaces decreases towards the distal end of the tip, with a maximum width at the region of the screw engaging configuration furthest away from the end tip.

According to the embodiment shown in Fig. 14C the width of each of the flattened external surfaces increases towards the distal end of the end tip, with a maximum width at the region where the longitudinal ribs reach the pointy rounded surface of the end tip.

According to the embodiment shown in Figs. 14D and 14E the width of each of the flattened external surfaces first increases and later maintains a constant width towards the distal end of the tip.

According to the embodiment shown in Fig. 14A the width of each of the flattened external surfaces firstly decreases, secondly increases, and later decreases again towards the distal end of the end tip.

The exposed central portion 13 of the arched convex surfaces, and optionally also of the arched concave surfaces, may have a constant width along the screw engaging configuration and even along the slender portion 14, providing a constant amount of friction retention between the screw engaging configuration and the driver engaging configuration, whatever the region of the exposed central portion 13 contacting with the driver engaging configuration.

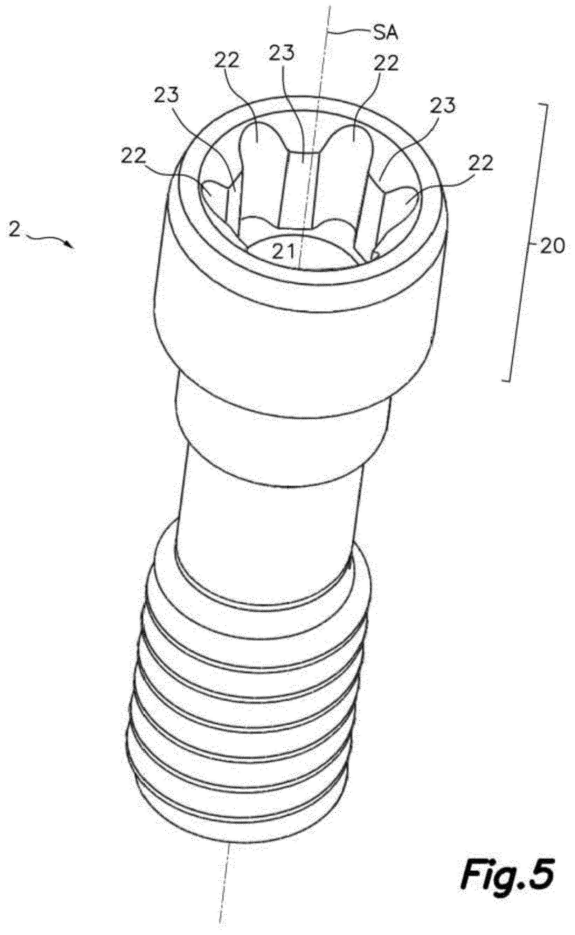

The proposed screw, shown in Fig. 5, comprises an elongated screw body, elongated in a longitudinal screw axis SA direction, with a driver engaging configuration 20 on a proximal end thereof, and with a threaded region on a distal end thereof.

The driver engaging configuration 20 will comprise a central socket 21, aligned with the longitudinal screw axis SA, surrounded by a number of longitudinal grooves 22 elongated in the direction of the longitudinal screw axis SA. Those longitudinal grooves 22 are radially distributed around the central socket 21 in radial symmetry, defining a lobular driver engaging configuration 20.

Successive transversal cross-sections of the central socket 21, perpendicular to the longitudinal screw axis SA, have a regular polygonal shape with a diminishing size towards the proximal end of the screw 2, defining a tapering central socket 21.

Each longitudinal groove 22 is defined between two adjacent sides of the regular polygonal shape of the central socket 21, a central portion 23 of each side of the regular polygonal shape of the central socket 21 being exposed, accessible from the central socket 21.

Preferably, the size of the central socket 21 and/or of the longitudinal grooves 22 diminishes in a linear manner towards the proximal end of the screw 2. According to that, the exposed central portion 23 of each side of the central socket 21 is preferably a flat surface which define an angle regarding the longitudinal screw axis SA, preferably an angle equal or smaller than 18° or equal or smaller than 10°.

To obtain a coupling between the screw and the screwdriver described above, the screwdriver 1 and the screw 2 will have regular polygonal shaped transversal cross-sections with the same number of sides, in this example six sides.

Preferably, the longitudinal grooves 22 of the driver engaging configuration 20 are according to the standard ISO 10664, corresponding to the industry standard known as Torx, but the exposed central portions of each side of the central socket 21 will not be according to this standard but will be flat surfaces, the Torx standard being contained within the perimeter of the driver engaging configuration 20.

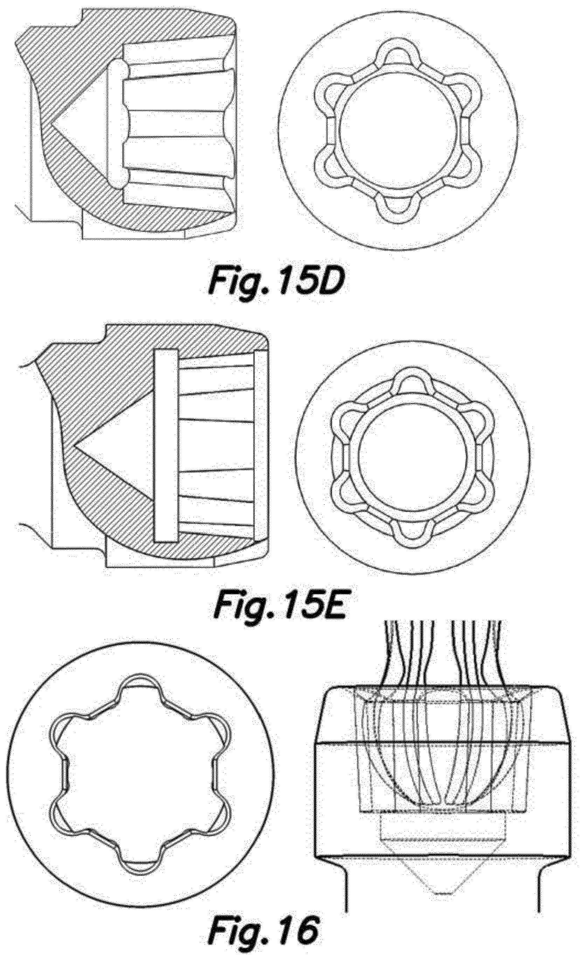

This feature allows the same driver engaging configuration 20 to be engageable by the proposed screw engaging configuration 10, as shown in Fig. 16, and also by other different screwdrivers currently commonly used in the industry.

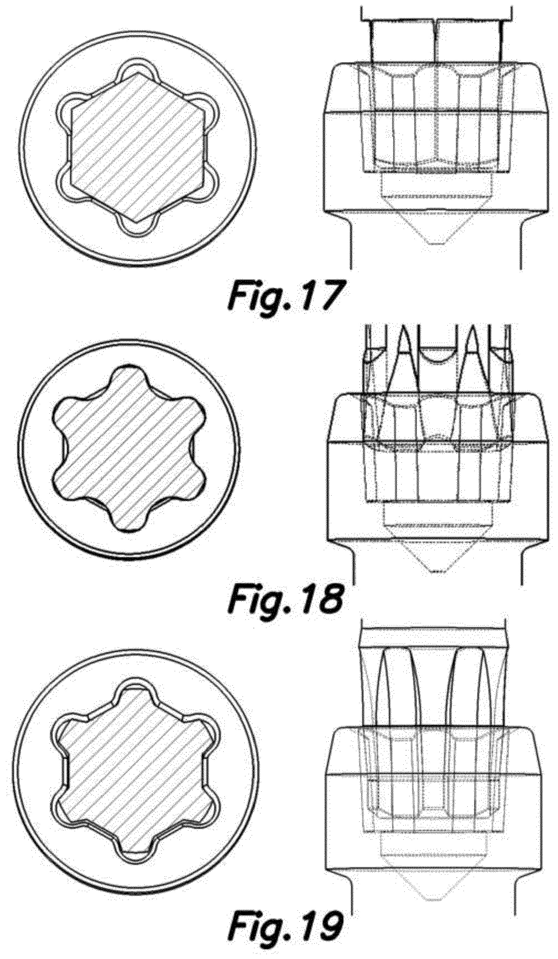

For example, the proposed screw can be coupled to a straight hexagonal screwdriver under the standard of the dental industry known as hex. E/C, which lacks protruding ribs, which gets engaged with the exposed central portions 23 of the central socket 21 without engaging with the longitudinal grooves 22, as shown in Fig. 17.

The proposed screw can also be engaged with a straight hexagonal screwdriver under the standard of the dental industry known as Torx, as shown in Fig. 18, which includes protruding ribs but lacks arched convex surfaces with a straight cross-section between the protruding ribs, which engages with the longitudinal grooves 22 without engaging with the exposed central portions 23 of the central socket 21.

According to an additional example, the proposed screw can be also engaged with a straight hexagonal screwdriver under the standard of the dental industry known as six node security, as shown in Fig. 19, which includes protruding ribs and arched concave surfaces with a straight cross-section between the protruding ribs.

None of the known screwdrivers according to the industry standards described above includes protruding ribs with arched convex surfaces in between, allowing for engagement with friction-retention and torque transmission between the driver engaging configuration and the screw engaging configuration at different angles, but all of them can be used to actuate the proposed screw with different limitations compared to the proposed screwdriver. According to that, the proposed screw is a multi-compatible screw which provides different features depending on which screwdriver is used therewith.

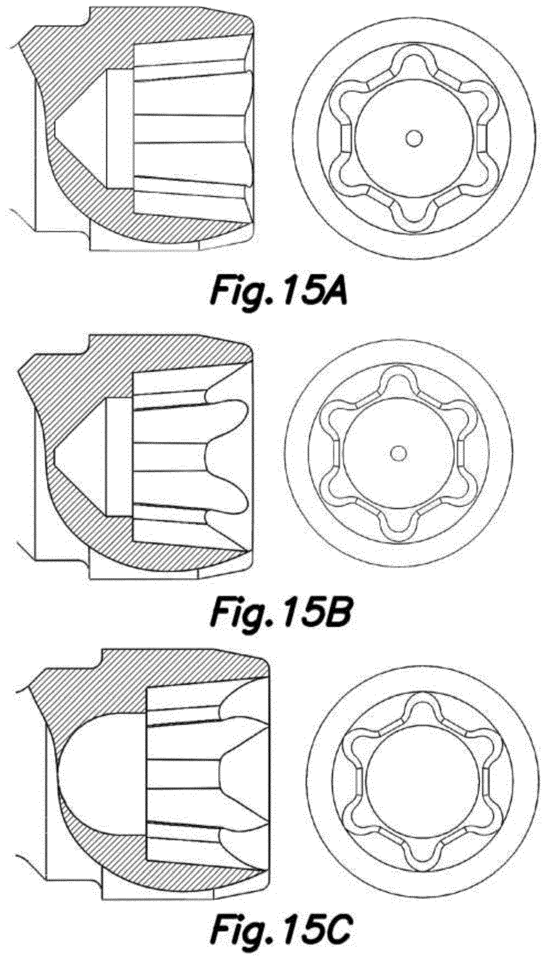

Both the mouth of the driver engaging configuration and the bottom of the central socket, opposed to said mouth, may have different configurations, providing different coupling angles between the screw and the screwdriver, self-guiding engagement, production costs, and other improvements. For example, Figs. 15A, 15B and 15C show different socket mouth embodiments.

The mouth of the driver engaging configuration shown in Figs. 15A and 15D is almost flat with a very shy conic concave shape towards the interior of the socket, for example with an angle bigger than 80° regarding the longitudinal screw axis SA.

The mouth of the driver engaging configuration shown in Fig. 15B have a clearly conic concave shape towards the interior of the socket, for example with an angle between 40° and 60° regarding the longitudinal screw axis SA

The mouth of the driver engaging configuration shown in Fig. 15C have a clearly hemi-spherical concave shape towards the interior of the socket, for example with an angle between 40° and 60° regarding the longitudinal screw axis SA.

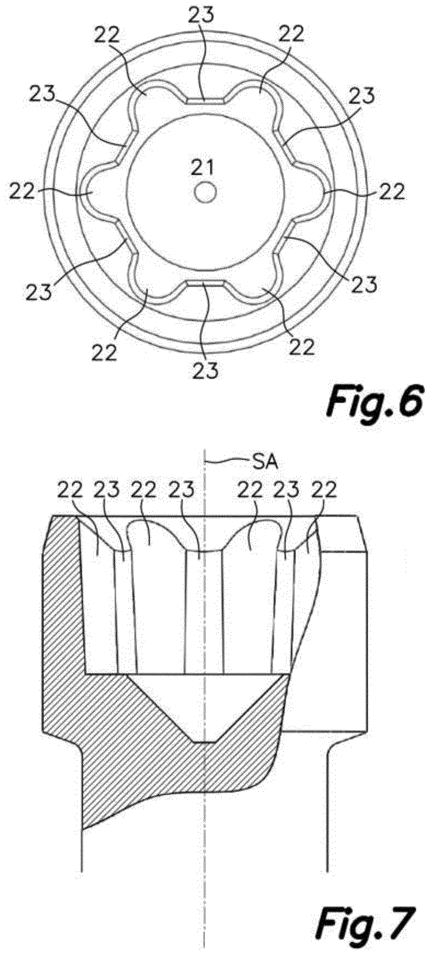

The central socket bottom shown in Figs. 7, 15A, 15B, 15D and 15E have a conical concave shape with a maximum diameter inscribed within the central socket 21.

The central socket bottom shown in Fig. 15C has a semi-spherical concave shape with a maximum diameter inscribed within the central socket 21.

Also, according to the embodiments shown in Figs. 15D and 15E, between the central socket, surrounded by the longitudinal grooves, and the conical or hemi-spherical concave shape of the central socket bottom, an annular groove, concentric with the screw longitudinal axis SA and with a maximal diameter bigger than the central socket, may interrupt the central portions 23, defined between the longitudinal grooves, at a region furthest away from the mouth of the driver engaging configuration.

The screw 2 and the screwdriver 1 described above constitute a screw fastening set.

To obtain a friction-retention between the screw and driver engaging configurations 10, 20, which keeps the screw attached to the screwdriver, the size of the biggest transversal cross-sections of the regular polygonal shaped core 11 of the screw engaging configuration 10 has to be defined between the biggest and the smallest sizes of the successive transversal cross-sections of the tapering central socket 21 so, when the screw engaging configuration 10 is inserted in the driver engaging configuration 20, the exposed central portions 13 of the arched convex surfaces of the core 11 are wedged between the convergent central portions 23 of the sides of the central socket 21, providing the friction-retention of the screw 2.

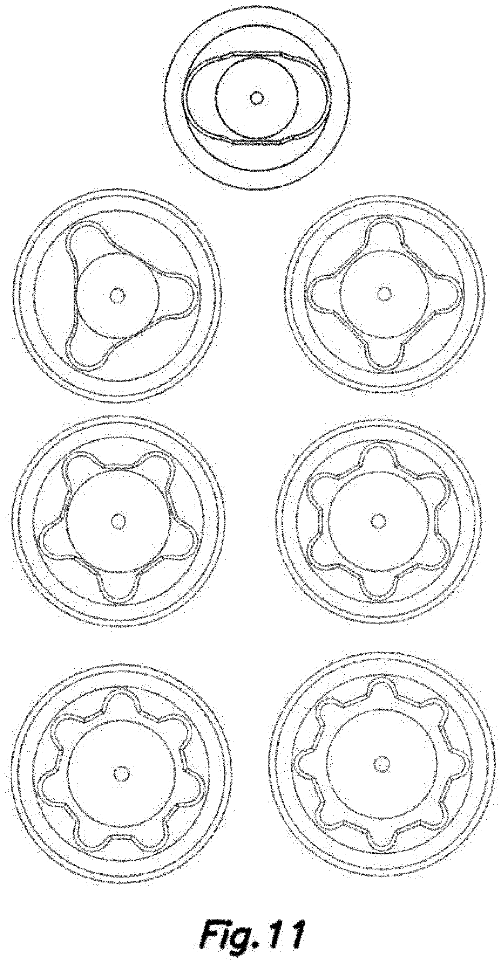

Preferably, the regular polygonal shaped transversal cross-sections of each the screwdriver 1 and of the screw 2 have at least four sides, but other embodiments are also contemplated, such those shown in Fig. 11, with an even or odd number of longitudinal ribs 12 and longitudinal grooves 22.

## Claims

1. A screwdriver for driving screws, the screwdriver (1) comprising an elongated driver body, elongated in a longitudinal driver axis (DA) direction, with a screw engaging configuration (10) on a distal end thereof, the screw engaging configuration (10) comprising:
a core (11) surrounded by a number of protruding longitudinal ribs (12) elongated in the longitudinal driver axis (DA) direction and radially distributed around the core (11), defining a lobular screw engaging configuration (10);
**characterized in that**
successive transversal cross-sections of the core (11), perpendicular to the longitudinal driver axis (DA), have a regular polygonal shape, and longitudinal cross-sections of the core (11), coincident with the longitudinal driver axis (DA), have a convex shape, defining a core (11) with several adjacent arched convex surfaces connected through arched edges,
each protruding longitudinal rib (12) is connected to the core (11) on an arched edge between two adjacent arched convex surfaces of the regular polygonal shape, or on one arched convex surface of the core (11), a central portion (13) of at least some of the arched convex surfaces of the core (11) being exposed.

2. The screwdriver according to claim 1 wherein the longitudinal cross-sections of the longitudinal ribs (12) protruding from the core (11), coincident with the longitudinal driver axis (DA), have a convex shape.

3. The screwdriver according to claim 1 or 2 wherein the convex shape of the longitudinal cross-section of the core (11), and/or of the longitudinal cross-section of the longitudinal ribs (12) protruding from the core (11), is/are substantially semicircular shaped and/or substantially pointed arch shaped.

4. The screwdriver according to claim 1, 2 or 3 wherein each arched convex surface of the core (11) is defined by a surface of zero Gaussian curvature.

5. The screwdriver according to any preceding claim wherein the core (11) is connected to the elongated driver body through a slender portion (14) thinner than the core (11), successive transversal cross-sections of the slender portion (14), perpendicular to the longitudinal driver axis (DA), having the same regular polygonal shape than the core (11), and longitudinal cross-sections of the slender portion (14), coincident with the longitudinal driver axis (DA), having a concave shape, defining a slender portion (14) with several adjacent arched concave surfaces.

6. The screwdriver according to claim 5 wherein the longitudinal ribs (12) also protrude from the slender portion (14), the longitudinal cross-section of the longitudinal ribs (12) protruding from the slender portion (14) being concave shaped.

7. The screwdriver according to any preceding claim wherein each longitudinal rib (12) is bordered by two longitudinal grooves (15) engraved in the core (11), the exposed central portion (13) of the arched convex surfaces of the core (11) being delimited between two of said longitudinal grooves (15).

8. The screwdriver according to any preceding claim wherein the screw engaging configuration (10) has at a distal end tip thereof a flattened end devoid of elongated ribs (12).

9. The screwdriver according to any preceding claim wherein the height of the longitudinal ribs (12) from the core (11) diminishes towards the distal end tip of the screwdriver (1) and/or wherein the width of the longitudinal ribs (12) diminishes towards the distal end tip of the screwdriver (1) and/or wherein the exposed central portion of the arched convex surfaces and optionally also of the arched concave surfaces have a constant width.

10. A screw comprising an elongated screw body, elongated in a longitudinal screw axis (SA) direction, with a driver engaging configuration (20) on a proximal end thereof, and with a threaded region on a distal end thereof, the driver engaging configuration (20) comprising:
a central socket (21) surrounded by a number of longitudinal grooves (22) elongated in the longitudinal screw axis (SA) direction and radially distributed around the central socket (21) in radial symmetry, defining a lobular driver engaging configuration (20);
**characterized in that**
successive transversal cross-sections of the central socket (21), perpendicular to the longitudinal screw axis (SA), have a regular polygonal shape with a diminishing size towards the proximal end of the screw (2), defining a tapering polygonal;
each longitudinal groove (22) is defined between two adjacent surfaces of the tapering polygonal socket, or on one of said surfaces of the tapering central socket, a central portion (23) of each of said surfaces of the tapering central socket being exposed.

11. The screw according to claim 10 wherein the longitudinal grooves (22) of the lobular driver engaging configuration are according to the standard ISO 10664.

12. The screw according to claim 10 wherein the size of the central socket (21) and/or of the longitudinal grooves (22) diminishes in a linear manner towards the proximal end of the screw (2).

13. The screw according to claim 11 or 12 wherein the exposed central portion (23) of each side of the central socket (21) defines an angle equal or smaller than 18° or equal or smaller than 10° regarding the longitudinal screw axis (SA) direction.

14. A screw fastening set comprising a screwdriver (1) according to any of the preceding claims 1 to 9 and a screw (2) according to any of the preceding claims 10 to 13, **characterized in that**:
the screwdriver (1) and the screw (2) have regular polygonal shaped transversal cross-sections with the same number of sides; and
the size of the biggest transversal cross-sections of the regular polygonal shaped core (11) of the screw engaging configuration (10) is between the biggest and the smallest sizes of the successive transversal cross-sections of the tapering central socket (21), providing friction-retention between the screw and driver engaging configurations (10, 20).

15. The screw fastening set according to claim 14 wherein the regular polygonal shaped transversal cross-sections of each the screwdriver (1) and of the screw (2) have at least four sides.
